# EUROPEAN PATENT SPECIFICATION

(11) **EP 0 557 319 B1**
(45) Date of publication and mention of the grant of the patent: **31.07.1996**
(21) Application number: 91918997.7
(22) Date of filing: 04.11.1991
(51) Int. Cl.: A61K 38/18

(54) **THERAPEUTIC USE OF FIBROBLAST GROWTH FACTOR**
THERAPEUTISCHE VERWENDUNG DES FIBROBLASTENWACHSTUMSFAKTORS
UTILISATION THERAPEUTIQUE DU FACTEUR DE CROISSANCE DE FIBROBLASTE

(30) Priority: 15.11.1990 GB 9024895
(43) Date of publication of application: 01.09.1993
(73) Proprietor: PHARMACIA S.p.A., 20152 Milano (IT)
(72) Inventor: CARMINATI, Paolo, I-20131 Milan (IT); MAZU , Guy, I-20148 Milan (IT)
(74) Representative: Ferrario, Vittorino
(86) International application number: EP9102078
(87) International publication number: WO9208473

## Description

The present invention relates to a fibroblast growth factor for use as hypotensive agent.

Fibroblast Growth Factors (FGFs) are a family of related proteins including basic Fibroblast Growth Factor (bFGF) and acidic Fibroblast Growth Factor (aFGF); aFGF and bFGF were originally purified to homogeneity from bovine brain (Thomas KA, Rios-Candelore M., Fitzpatrick S. Purification and characterization of acidic fibroblast growth factor from bovine brain. Proc. Natl. Acad. Sci USA 1984; 81:357-61) and from bovine pituitary (Gospodarowicz D. Purification of a fibroblast growth factor from bovine pituitary. J. Biol. Chem. 1975; 250; 2515-20) respectively.

FGFs have been shown to have a wide spectrum of biological activities both in vitro and in vivo (European Journal of Clinical Investigation 1988; 18, 321-336).

They are best known as endothelial cell mitogens, but they stimulate virtually all mesoderm- and neuroectoderm- derived cells.

Recent studies show that FGFs also stimulates melanocytes (Halaban R., Ghosh S., Baird A. bFGF is the putative natural growth factor for human melanocytes. In Vitro 1987; 23: 47-52), and induce anchorage- independent growth of certain established fibroblast lines (Huang SS, Kuo M-D, Huang JS. Transforming growth factor activity of bovine brain-derived growth factor. Biochem. Biophys. Res. Comm. 1986; 139:619-25; Rizzino A., Ruff E. Fibroblast growth factor induces the soft agar growth of two non-transformed cell lines. In Vitro 1986; 22:749-56).

FGFs also possess a number of non mitogenic functions in vitro, including chemotaxis (Connolly DT, Stoddard BL, Harakas NK, Feder. J. Human fibroblast-derived growth factor is mitogen and chemoattractant for endothelial cells. Biochem. Biophys. Res. Comm. 1987; 144:705-12; Senior RM, Huang SS, Griffin GL, Huang JS. Brain-derived growth factor is a chemoattractant for fibroblast and astroglial cells. Biochem. Biophys. Res. Comm. 1986; 141:67-72), protease induction (Moscatelli D, Presta M. Rifkin DB. Purification of a factor from human placenta that stimulates capillary endothelial cell protease production, DNA synthesis and migration. Proc. Natl. Acad. Sci USA 1986; 83:2091-5; Phadke K. Fibroblast growth factor enhances the interleukin-1-mediated chondrocytic protease release. Biochem. Biophys. Res. Comm. 1987; 142; 448-53), neurite outgrowth and neuronal cell survival (Togari A, Baker D, Dickens G, Guroff G. The neurite promoting effect of fibroblast growth factor on PC12 cells. Biochem. Biophys. Res. Comm. 1983; 114:1189-93; Wagner JA, D'Amore P. Neurite outgrowth induced by an endothelial cell mitogen isolated from retina. J. Cell Biol: 1986; 103:1363-7; Walicke P, Cowan WM, Ueno N, Baird A. Guillemin R. Fibroblast growth factor promotes survival of dissociated hippocampal neurons and enhances neurite extension. Proc. Natl. Acad. Sci USA 1986; 83:3012-6; Morrison RS, Sharma A, De Vellis J, Bradshaw RA. Basic fibroblast growth factor supports the survival of cerebral cortical neurons in primary culture. Proc. Natl. Acad. Sci. USA 1986;83:7537-41; Schubert D, Ling N, Baird A. Multiple influences of a heparin-binding growth factor on neuronal development. J. Cell Biol. 1987;104:635-43), muscle cell differentiation (Lathrop B, Oslon E, Glaser L. Control by fibrobalst growth factor of differentiation in the BC3H1 muscle cell line. J. Cell Biol. 1985;100:1540-9), and various endocrine functions (Gospedarowicz D, Ferrara N, Schweigerer L, Neufeld G. Structural characterization and biological functions of fibroblast growth factor. Endocr. Rev. 1987;8:95-114; Baird A, Mormede P, Ying S-Y et al. A nonmitogenic pituitary function of fibroblast growth factor: regulation of thyrotropin and prolactin secretion. Proc. Natl. Acad. Sci USA 1985; 82:5545-9; Baird A, Hsueh AJW. Fibroblast growth factor as an intraovarian hormone: Differential regulation of steroidogenesis by an angiogenic factor. Reg. Peptides 1986;16:243-50).

In vivo FGFs are best known for their ability to stimulate neovascularization (Gospodarowicz D, Ferrara N, Schweigerer L, Neufeld G. Structural characterization and biological functions of fibroblast growth factor. Endocr. Rev. 1987;8:95-114). They also induce limb regeneration in lower vertebrates (Gospodarowicz D, Ferrara N, Schweigerer L, Neufeld G. Structural characterization and biological functions of fibroblast growth factor. Endocr. Rev. 1987;8:95-114) and stimulate wound healing (Buntrock P, Jentzsch KD, Heder G. Stimulation of wound healing using brain extract with fibroblast growth factor (FGF) activity. Exp. Pathol 1982;21:62-7; Thomas KA, Gimenez-Gallego G, Di Salvo J et al. Structure and activities of acidic fibroblast growth factor. In: Rifkin DB, Klagsburn M, eds. Angiogenesis. New York: Cold Spring Harbor, 1987;9-12). Recent studies point to novel in vivo roles for FGFs: Slack and co-workers (Slack JMW, Darlington BG, Heath JK, Godsave SF. Mesoderm induction in early Xenopus embryos by heparin-binding growth factors. Nature 1987;326:197-200) have shown that both bFGF and aFGF mimic the ventrovegetal signal leading to the induction of ventral-type mesoderm in the Xenopus blastula.

The variety of in vitro and in vivo functions of FGFs, as well as their wide tissue distribution, suggests that these mitogens are of broad physiological significance and of potential clinical value.

Among the many possible clinical uses, FGFs can find an application in the healing of wounds, ulcers and bums, in the revascularization of ischaemic tissues, in the regeneration of damaged neural tissue or as adjunct to other therapeutic regimens for example during transplantation.

Despite the wide range of in vitro and in vivo activities attributed to FGFs, the prior art does not describe any activity in the lowering of blood pressure.

We have now surprisingly found that the systemic administration of low concentrations of polypeptides belonging to the FGF family, in particular bFGF, induce in vivo a considerable vasodilatory effect.

These findings are of major importance in the therapeutical application of the FGFs, particularly bFGF, as anti-hypertensive agents for example in the prevention of acute myocardial infarction and of cerebral hemorrhages.

The present invention relates to a fibroblast growth factor (FGF) for use as hypotensive agent.

The present invention thus provides the use of fibroblast growth factor for the manufacture of a hypotensive agent for the treatment or prophylaxis of a mammal, including man.

The invention further relates to an agent for use as a hypotensive compound comprising fibroblast growth factor.

Further, the invention has utility in providing a method of reducing blood pressure in a mammal, including man, by administering an effective amount of fibroblast growth factor to a recipient in need of such therapy.

In this Application, FGF includes the class of polypeptides that have biological activity similar to that exhibited by the natural human FGF polypeptide. Thus, FGF includes acidic and basic FGF, such as human FGF produced by recombinant DNA techniques or derived from natural sources, as well as closely related mammalian FGF, e.g. bovine, murine or rodent. Any bFGF molecule as described in, for instance, WO 86/07595; WO 87/01728; EP-A-0226181; Abraham et al, EMBO J. 5, 2523-2528, 1986; or Lobb, Eur. J. Clin. Invest. 18, 321-336, 1988; may be usefully employed in the invention. In particular human and bovine bFGF according to the present invention constitute the preferred compounds.

Such factors, i.e. human and bovine bFGF and human and bovine aFGF, are well known: they are described, for example, in the published International Patent Applications PCT WO86/07595 and PCT WO87/01728, and in the published European Patent Applications no. 226181 and no. 237966, as well as in many scientific publications such as, for example, Science vol. 233, pp. 545-548, August 1, 1986; Embo Journal vol. 5, no. 10, pp. 2523-2528, 1986.

Human bFGF is a 146 amino acid long polypeptide having the following amino acid sequence

1                                   10
Pro Ala Leu Pro Glu Asp Gly Gly Ser Gly Ala Phe Pro Pro Gly His
              20                                       30
Phe Lys Asp Pro Lys Arg Leu Tyr Cys Lys Asn Gly Gly Phe Phe Leu
                              40
Arg Ile His Pro Asp Gly Arg Val Asp Gly Val Arg Glu Lys Ser Asp
    50                                        60
Pro His Ile Lys Leu Gln Leu Gln Ala Glu Glu Arg Gly Val Val Ser
                      70                                       80
Ile Lys Gly Val Cys Ala Asn Arg Tyr Leu Ala Met Lys Glu Asp Gly
                                      90
Arg Leu Leu Ala Ser Lys Cys Val Thr Asp Glu Cys Phe Phe Phe Glu
              100                                      110
Arg Leu Gly Ser Asn Asn Tyr Asn Thr Tyr Arg Ser Arg Lys Tyr Thr
                              120
Ser Trp Tyr Val Ala Leu Lys Arg Thr Gly Gln Tyr Lys Leu Gly Ser
    130                                       140
Lys Thr Gly Pro Gly Gln Lys Ala Ile Leu Phe Leu Pro Met Ser Ala
    146
Lys Ser

Bovine bFGF differs from the human analogue in position 112, where threonine is substituted by serine, and in position 128, where serine is substituted by proline.

Both human and bovine bFGF may present an N-terminal extension including the whole or a part of the following 11 amino acid sequence
i) Gly-Thr-Met-Ala-Ala-Gly-Ser-Ile-Thr-Thr-Leu
   for example, in particular, the following 9 amino acids:
ii) Met-Ala-Ala-Gly-Ser-Ile-Thr-Thr-Leu,
   or the following 8 amino acids
iii) Ala-Ala-Gly-Ser-Ile-Thr-Thr-Leu
   or the following 7 amino acids
iv) Ala-Gly-Ser-Ile-Thr-Thr-Leu.

Even shorter forms of the 146 amino acid long molecules were isolated; these are N-terminal deleted bFGFs lacking of one or more amino acid residues.

All the FGFs of the invention may also be amidated at their C-terminal end.

FGFs analogues with comparable biological activities, in particular with similar capability to lower the blood pressure, are also included in the scope of the invention.

An analogue may be represented by a mutein derived from the above indicated FGFs, either amidated or not, characterized in that its amino acid sequence differs from the sequence of a naturally occurring FGF by way of amino acid substitutions, deletions, inversion and/or additions. Examples of basic FGF analogues may be found in the published European Patent Application no. 363675.

Also mixtures of two or more different FGFs are to be considered within the scope of the present invention.

In particular, for example, a mixture of two different forms of human bFGF having a N-terminal extension of 7 and 8 amino acid residues respectively with respect to the natural 146 amino acid long molecule, represents one preferred embodiment of the present invention.

The detailed amino acid sequence of these two forms is that reported for human bFGF on page 6 and having the N-terminal extensions indicated on page 7 under point iii) and point iv) respectively.

In this specification this mixture is indicated with the name FCE 26184. FCE 26184 is available from Farmitalia Carlo Erba Biotechnology Development Department.

Thus, the term "fibroblast growth factor" as used in the present patent application includes the natural proteins as well as the above indicated analogues and mixtures.

The FGFs of the invention are generally obtained by recombinant DNA techniques with well known procedures such as those described in the previously mentioned International Patent Applications No. PCT/WO86/07595 and PCT/WO87/01728 or in the European Patent Applications No. 226181, 237966 and 363675.

The hypotensive activity of the FGFs of the present invention, was shown in a series of experiments carried out on animals anesthesized with a mixture of ketamine, diazepam and atropine according to the conditions depicted below in the experimental section. A similar hypotensive effect can be recorded also in non-anesthesized animals.

Several animal species including rats and rabbits, especially rats, could be used as experimental models.

For the experiments a buffered solution of FGF is injected as a bolus into the jugular vein of the rat; the left femoral artery is cannulated and the catheter is placed into the abdominal aorta for continuous recording of the blood pressure; the injection can also be carried out into the femoral vein and/or in contralateral common femoral artery under similar experimental conditions giving comparable results.

Thus for the compound FCE 26184 the hypotensive effect in rat was evident 1 or 2 minutes after the injection and it was recorded as a decrease in blood pressure starting from a basal level of about 70 mm of Hg down to about 25 mm of Hg.

The animals were given increasing anounts of FGF (FCE 26184) and it was demonstrated that the hypotensive effect was dose-dependent for doses ranging from about 1.25 µg/kg of body weight to about 2.75 µg/kg of body weight; the effect saturated beyond this dose while it was practically undetectable below 1.25 µg/kg of body weight.

Similar results could be obtained also with the other FGF related molecules according to the invention and, in any case, the obtained results were independent of the presence or absence in the injected solution of sulphated polysaccharides (i.e. heparin) which stabilizes the protein.

In view of their hypotensive activity, the fibroblast growth factors of the invention can find useful application as, e.g. anti-hypertensive agents for instance in the prevention and treatment of hypertension related pathologies, e.g. in situations like acute myocardial infarction and cerebral hemorrhages.

The FGFs of the invention can be administered in form of pharmaceutical compositions comprising one or more of said FGFs, as such or as pharmaceutically acceptable salts together with one or more pharmaceutically acceptable excipients, e.g. carriers and/or diluents.

The pharmaceutically acceptable salts may be the salts of pharmaceutically acceptable inorganic acids, e.g. hydrochloric, hydrobromic, sulphuric and phosphoric acid, or pharmaceutically acceptable organic acids, e.g. acetic, citric, maleic, malic, succinic and tartaric acid.

A preferred way of administration is by bolus injection of a phosphate buffered solution.

The pharmaceutical compositions of the invention can be prepared with known techniques according to usual galenic procedures.

The dose to be administered will depend on the specific pathological condition to be treated and on the desired entity of the decrease in blood pressure.

In general, the FGFs of the invention can be administered at doses ranging from 0,2 to 20 µg/kg of body weight.

In the accompanying Figure 1 the sequence of the 155 amino acid long form of human bFGF is represented.

The present invention will be further illustrated by the following examples.

### Preparation examples

### Preparation of FCE 26184.

The construction of the synthetic DNA sequence for bFGF and of the expression plasmid carrying such sequence was performed according to the procedure described in EP 363675. The fermentation and purification process was carried out as follows:

### (a) Fermentation process

A bacterial strain, E. coli type B, from the Institute Pasteur collection, was transformed with a plasmid carrying both the human gene coding for bFGF and the gene for tetracycline resistance. This transformed strain was used for the production of recombinant non-glycosylated h-bFGF (human bFGF). A Master Cell Bank (15 freeze-dried vials) and a Working Cell Bank (W.C.B.) (70 vials stored in liquid nitrogen at -190°C) of this strain were prepared. The content of one vial of W.C.B. was used as the inoculum for the fermentation phase.

The fermentation process was carried out in 10 l fermentors filled with 4 l of culture medium.

Tetracycline hydrochloride was added to the medium in order to maintain the conditions of strain selection.

After 20 hours of growth at 37°C the final biomass was 42 ± 2 g/l dry weight, and the production of bFGF was 2500 ± 500 mg/l as measured by comparative gel electrophoresis.

Enrichment in pure oxygen was required during the fermentation phase in order to allow a large bacterial growth.

### (b) Initial purification

The cells (microorganisms) were separated from the total fermentation broth by centrifugation. The resulting pellet was resuspended in a sodium phosphate buffer containing sodium chloride. A minimum of 3 passages through a high pressure homogenizer were necessary for efficient cell breakage. The resulting cell lysate was clarified by centrifugation and the supernatant was collected for further processing.

### (c) Purification

The clarified supernatant was loaded on a column of Sepharose (Trade Mark) S Fast Flow (cation exchanger) and the product was eluted from this column using a gradient of increasing sodium chloride concentrations in a phosphate buffer (Trade Mark). The product was further purified on a column of Heparin Sepharose 6 B by eluting with a gradient of increasing sodium chloride concentration in a phosphate buffer. Finally a buffer exchange was made on a Sephadex (Trade Mark) G25 resin to obtain the product in the bulk product buffer (Sodium phosphate -EDTA).

### (d) Colum sanitization

Sepharose S Fast Flow and Sephadex G25 columns were sanitized by washing with sodium hydroxide solutions. Heparin Sepharose was washed alternatively with solutions at pH = 8.5 and pH = 5.5 containing 3M sodium chloride.

In this way, a 154/153 form of bFGF was obtained,.

This is an approximately 50:50 mixture of:
- a 154 amino acid human bFGF [(2-155)bFGF] having the amino acid squence of the 155 amino acid form which is reported by Abraham et al but without the N-terminal Met residue, this 154 amino acid sequence also being shown in Figure 1 of the accompanying drawings; and
- a 153 amino acid human bFGF [(3-155)bFGF] consisting of the above 154 amino acid form without the Ala residue at position 1.

### EXAMPLE 1

Vascular response to recombinant human basic Fibrobalst Growth Factor (FCE 26184) in rats.

### 1. Animals

The experiments were performed on 11 Wistar female rats weighing between 150 and 200 g. The animals were housed in cages and the room was lighted in 12 hour cycles; food and water were available ad libitum.

### 2. Surgical preparation

The rats were anesthetized with 0.8 ml i.p. of a mixture of 25 mg/ml Ketamine hydrochloride, 2mg/ml Diazepam and 0.1 mg/ml Atropine. The left femoral artery was cannulated with a polythene tubing and a catheter was retrogradely placed into the abdominal aorta for the measurements of central blood pressure. To prevent the thrombotic occlusion the aortic cannula was filled with a heparin solution and was connected to a pressure transducer (Model P23XL, Spectramed Inc. Oxnard CA) linked in turn to a calibrated polygraph (Pelker-Elmer 56). Mean arterial blood pressure (MAP) was recorded continuously during the experiments.

### 3. FCE 26184 injection

As stated above, FCE 26814 represents a recombinant human bFGF having an N-terminal extension of 7-8 amino acid residues. This compound is therefore a mixture of two bFGF forms with a length of 153-154 amino acids. A solution of FCE 26814 (batch OP48) in phosphate buffered saline containing 0.1% sodium heparin was administered to the rats in a single 50 µl bolus injection into the jugular vein, 5 minutes after baseline evaluation. Each animal received the following doses of the compound:
190 ng, 247 ng, 323 ng, 421 ng, 550 ng, 2600 ng. Two animals received each of the above doses, except that the dose of 190 ng was given to one rat.

### 4, Results

Table 1 shows that systemic treatment with basic FGF (FCE 26184) induces a decrease in arterial blood pressure. This effect was recorded starting after 1-2 min. from the time of injection. The effect was evident at a dose of 247 ng/rat (1.25 µg/kg) and reached a maximal response at 550 ng/rat (2.75 µg/kg). No further drops in blood pressure was observed by increasing the bFGF dose up to 2600 ng/rat (13 µg/kg)

**TABLE 1**

| Amount of bFGF administered per rat | Arterial blood pressure (mm Hg) |
|---|---|
| 0 ng | 70 |
| 190 ng | 70 |
| 247 ng | 60 |
| 323 ng | 44 |
| 421 ng | 30 |
| 421 ng | 25 |
| 2600 ng | 25 |

### Conclusion

bFGF (FCE 26184) given intravenously to rats decreased arterial blood pressure starting from the dose of 1,25 µg/kg. This effect was highest at 2.75 µg/kg and saturated at higher concentrations.

1                                   10

Met Ala Ala Gly Ser Ile Thr Thr Leu Pro Ala Leu Pro Glu Asp Gly

              20                                        30

Gly Ser Gly Ala Phe Pro Pro Gly His Phe Lys Asp Pro Lys Arg Leu

                              40

Tyr Cys Lys Asn Gly Gly Phe Phe Leu Arg Ile His Pro Asp Gly Arg

      50                                        60

Val Asp Gly Val Arg Glu Lys Ser Asp Pro His Ile Lys Leu Gln Leu

                      70                                        80

Gln Ala Glu Glu Arg Gly Val Val Ser Ile Lys Gly Val Cys Ala Asn

                                      90

Arg Tyr Leu Ala Met Lys Glu Asp Gly Arg Leu Leu Ala Ser Lys Cys

              100                                       110

Val Thr Asp Glu Cys Phe Phe Phe Glu Arg Leu Gly Ser Asn Asn Tyr

                              120

Asn Thr Tyr Arg Ser Arg Lys Tyr Thr Ser Trp Tyr Val Ala Leu Lys

      130                                       140

Arg Thr Gly Gln Tyr Lys Leu Gly Ser Lys Thr Gly Pro Gly Gln Lys

                      150                 155

Ala Ile Leu Phe Leu Pro Met Ser Ala Lys Ser

## Claims

1. Use of Fibroblast Growth Factor (FGF) for the manufacture of a hypotensive agent for the treatment or prophylaxis of a mammal, including man.

2. Use of a Fibroblast Growth Factor according to claim 1 wherein said factor is a basic Fibroblast Growth Factor (bFGF).

3. Use of a Fibroblast Growth Factor according to claim 2 wherein said factor is human bFGF or an analogue thereof.

4. Use of a Fibroblast Growth Factor according to claim 2 wherein said factor is bovine bFGF or an analogue thereof.

5. Use of a Fibroblast Growth Factor according to anyone of claims 2 to 4 wherein said factor is a mixture of two or more different forms of bFGF.

6. Use of a Fibroblast Growth Factor according to anyone of claims 3 to 5 wherein said analogue is a deletion mutant of the whole molecule.

## Patentansprüche

1. Verwendung von Fibroblast-Wachstumsfaktor (FGF) zur Herstellung eines blutdrucksenkenden Mittels zur Behandlung oder Prophylaxe von Säugern, einschliesslich Menschen.

2. Verwendung von Fibroblast-Wachstumsfaktor gemäss Anspruch 1, worin der Faktor ein basischer Fibroblast-Wachstumsfaktor (bFGF) ist.

3. Verwendung von Fibroblast-Wachstumsfaktor gemäss Anspruch 2, worin der Faktor ein Human-bFGF oder ein Analogon davon ist.

4. Verwendung von Fibroblast-Wachstumsfaktor gemäss Anspruch 2, worin der Faktor ein Rinder-bFGF oder ein Analogon davon ist.

5. Verwendung von Fibroblast-Wachstumsfaktor gemäss mindestens einem der Ansprüche 2 bis 4, worin der Faktor eine Mischung aus zwei oder mehr verschiedenen bFGF-Formen ist.

6. Verwendung von Fibroblast-Wachstumsfaktor gemäss mindestens einem der Ansprüche 3 bis 5, worin das Analogon eine Deletionsmutante des Gesamtmoleküls ist.

## Revendications

1. Emploi du Facteur de Croissance des Fibroblastes (FGF) pour la fabrication d'un agent hypotensif pour le traitment ou la prophylaxie d'un mammifère, y inclus l'homme.

2. Emploi d'un Facteur de Croissance des Fibroblastes selon la revendication 1, caractérisé par le fait que le facteur mentionné est un Facteur de Croissance des Fibroblastes basique (bFGF).

3. Emploi d'un Facteur de Croissance des Fibroblastes selon la revendication 2, caractérisé par le fait que le facteur mentionné est le bFGF humain ou un analogue de ce dernier.

4. Emploi d'un Facteur de Croissance des Fibroblastes selon la revendication 2, caractérisé par le fait que le facteur mentionné est le bFGF bovin ou un analogue de ce dernier.

5. Emploi d'un Facteur de Croissance des Fibroblastes selon n'importe quelle revendication de 2 à 4, caractérisé par le fait que le facteur mentionné est un mélange de deux ou plusieurs formes de bFGF.

6. Emploi d'un Facteur de Croissance des Fibroblastes selon n'importe quelle revendication de 3 à 5, caractérisé par le fait que cet analogues est un mutant par délétion de toute la molécule.
